# FASCICULE DE BREVET EUROPEEN

(11) **EP 0 370 873 B1**
(45) Date de publication et mention de la délivrance du brevet: **17.03.1993**
(21) Numéro de dépôt: 89403161.6
(22) Date de dépôt: 17.11.1989
(51) Int. Cl.: G01N 33/28

(54) **Méthode et dispositif d'analyse d'un fluide polyphasique en écoulement dans une conduite**
Verfahren und Einrichtung für die Analyse einer durch eine Leitung strömenden mehrphasigen Flüssigkeit
Method and apparatus for the analysis of a polyphasic fluid flowing through a conduit

(30) Priorité: 23.11.1988 FR 8815236
(43) Date de publication de la demande: 30.05.1990
(73) Titulaire: INSTITUT FRANCAIS DU PETROLE, 92502 Rueil-Malmaison (FR)
(72) Inventeur: Castel, Yvon, F-78290 Croissy sur Seine (FR)
(74) Mandataire: Andreeff, François

(56) Documents cités:
- FR-A- 1 599 037
- FR-A- 2 593 921
- US-A- 3 344 659
- US-A- 4 596 136
- US-A- 4 776 210

## Description

L'invention concerne une méthode et un dispositif d'analyse d'un fluide polyphasique en écoulement, notamment un effluent de puits pétrolier d'accès difficile, tel un puits sous-marin ou un puits situé dans la forêt vierge.

En général, les effluents des puits pétroliers comportent de l'eau, de l'huile et du gaz hydrocarboné dont chacun constitue une phase propre au sein du fluide en production. Les effluents pétroliers peuvent en outre comporter des matériaux solides, tels du sable.

Cependant, au cours de la production d'un puits, les volumes relatifs de chaque phase du fluide, ainsi que les caractéristiques physiques et/ou chimiques des composants de ces phases, telles que le débit des fluides, peuvent varier beaucoup dans le temps.

Pour ajuster les traitements du fluide ou vérifier leur bon déroulement, il est nécessaire de connaître les quantités et les caractéristiques des composants de ces phases. De tels traitements peuvent être de l'injection d'eau comportant des tensio-actifs ou des polymères. Par ailleurs, ces traitements peuvent comporter une injection de gaz (naturel ou CO₂) ou de vapeur. Ils peuvent être des traitements d'anticorrosion (ajout d'inhibiteur de corrosion), des traitements contre la formation d'hydrates, le dépôt de paraffines ou de sels. Ces traitements facilitent le transport du fluide ou la production des gisements pétroliers.

Ces caractéristiques sont notamment le débit de fluide produit par le puits, la répartition quantitative des phases de ce fluides, mais aussi, plus particulièrement, pour l'eau : la résistivité, la densité, le pH, la viscosité ; pour l'huile : la densité, la viscosité, la conductivité électrique, la conductibilité thermique ; ou pour le gaz ; la densité, la composition molaire, ou encore pour l'eau et l'huile : la tension interfaciale.

Le brevet français 1.599.037 décrit un procédé et un dispositif pour déterminer la composition des fluides produits par un puits de pétrole, utilisant une chambre de prélèvement d'un échantillon du fluide, cette chambre étant descendue dans le puits. L'enseignement de ce document présente notamment l'inconvénient de ne pas permettre de vérifier si l'échantillonnage réalisé est représentatif du fluide circulant en moyenne dans le puits, de ne fournir qu'un nombre très limité de mesures et d'être difficilement utilisable dans les milieux hostiles en particulier pour des puits sous-marins.

Pour remédier entre autres choses à ces inconvénients, et plus spécifiquement pour réaliser des analyses à la sortie d'un ou plusieurs puits, tels des puits pétroliers, ou sur des conduites véhiculant des fluides polyphasiques, la présente invention propose une méthode et un dispositif autonome et très fiable.

La méthode d'analyse d'un fluide polyphasique en écoulement dans une conduite, selon l'invention, se caractérise notamment en ce qu'on effectue les étapes suivantes :
- on dérive ledit écoulement vers une chambre de puisage pendant le temps nécessaire à un remplissage,
- la chambre de puisage étant remplie, on rétablit ledit écoulement dans ladite conduite,
- on produit une décantation du fluide situé dans ladite chambre, pendant un certain temps, jusqu'à obtenir une séparation des différentes phases qui composent le fluide,
- on détermine un paramètre représentatif du volume d'au moins une phase, en mesurant une caractéristique sensible à la variation de composition d'au moins deux phases dudit fluide, et
- on contrôle un paramètre macroscopique rendant compte de la qualité du fluide dans son ensemble pendant le remplissage de la chambre et avant et/ou après ledit remplissage de la chambre.

Ledit paramètre macroscopique du fluide pourra être la masse de fluide en écoulement située dans un tronçon dudit conduit, par exemple en utilisant une boucle de pesée.

On pourra mettre en oeuvre ladite méthode pour des vitesses différentes dudit écoulement. En effet, dans un puits pétrolier, par exemple, la répartition des phases ou des constituants des phases varie suivant la vitesse de l'écoulement de fluide.

On pourra prélever dans au moins deux phases, un échantillon de substance que l'on enverra successivement vers une série d'analyseurs par l'intermédiaire d'un circuit de prélèvement.

Comme certains échantillons peuvent laisser des dépôts, tels des asphaltènes, dans le circuit de prélèvement ou dans la série d'analyseurs, ces dépôts pouvant altérer les mesures ou boucher les circuits ou les analyseurs, on pourra avantageusement rincer le circuit de prélèvement et ladite série d'analyseurs en y faisant circuler de l'eau de mer. L'eau de mer pourra en outre servir au réétalonnage de la série d'analyseurs.

Pour améliorer ce rinçage ou diminuer la viscosité des échantillons, on pourra réchauffer ledit échantillon de substance prélevée et/ou l'eau de mer avant de les faire circuler dans la série d'analyseurs.

La connaissance de la production ou de ses variations d'un puits pétrolier en production est particulièrement intéressante dans l'exploitation d'un gisement, car elle permet, par exemple, de contrôler correctement la production d'un puits et d'assurer sa longévité. De même, pour plusieurs puits regroupés sur un collecteur, la connaissance du débit de chaque puits permet une optimisation de leur production. Aussi, il sera avantageux de mesurer le temps nécessaire pour remplir un volume connu du réservoir et ainsi en déduire le débit du fluide. Cette mesure trouve aussi son application dans le transport à distance des fluides polyphasiques en permettant de préparer à temps le matériel, tel des séparateurs, lorsque des changements dans la répartition des phases apparaissent, par exemple lors de la venue de bouchons de gaz.

Le dispositif d'analyse d'un fluide polyphasique en écoulement dans une conduite selon l'invention, est notamment caractérisé en ce qu'il comporte un détecteur d'un paramètre macroscopique rendant compte de la qualité du fluide en écoulement dans son ensemble, ledit détecteur étant disposé sur ladite conduite en amont d'une chambre de puisage adaptée à prélever une quantité du fluide circulant dans ladite conduite, ladite chambre comportant des moyens de mesure d'au moins un paramètre représentatif du volume d'au moins une phase, ladite conduite comportant des moyens permettant d'orienter le fluide dans ladite chambre.

Ledit détecteur de paramètre macroscopique de la qualité du fluide pourra être un détecteur de la masse de fluide en écoulement situé dans un tronçon de ladite conduite.

Le dispositif pourra comporter un bras mobile disposé à l'intérieur de ladite chambre et dont une extrémité est adaptée à balayer au moins une partie du volume de ladite chambre, l'extrémité du bras comportant un capteur de mesure d'une caractéristique du fluide. Ledit bras pourra être déplacé par un organe moteur, tel un vérin hydraulique.

Le capteur de mesure d'une caractéristique du fluide pourra avantageusement comporter une cellule générant des ultrasons. En effet, les fluides analysés peuvent produire des dépôts salissant le capteur et affectant les mesures de ces derniers.

L'usage dans un capteur d'une cellule générant des ultrasons, permet de nettoyer le capteur. A cette fin de nettoyer le capteur, on pourra avantageusement utiliser des capteurs à ultrasons, qui offrent en outre l'avantage de fournir des mesures utiles, telles que la vitesse du son dans un liquide ou dans un gaz, ou bien la masse volumique des composants d'une phase et peuvent permettre d'identifier les composants des phases.

Le dispositif pourra comporter un circuit de prélèvement fixé audit bras, de préférence à l'extrémité mobile du bras qui balaye une grande partie du volume du réservoir, ce circuit ayant une première extrémité débouchant au niveau du capteur de mesure et une deuxième extrémité alimentant une série d'analyseurs que l'on pourra placer à l'extérieur de la chambre de puisage, dans une enceinte étanche remplie de gaz, tel un gaz neutre, sensiblement à la pression atmosphérique.

Les moyens de mesure d'un paramètre représentatif du volume d'au moins une phase du fluide pourra comporter un capteur de mesure d'une caractéristique du fluide, tel ledit capteur à ultrasons, et un capteur de position du bras.

Le dispositif pourra comporter des moyens de chauffage disposés dans le circuit de prélèvement. Ces moyens de chauffage pourront être disposés dans l'enceinte.

La chambre de puisage pourra comporter une membrane souple délimitant au moins une partie de volume du fluide prélevé. De cette manière, il est possible de prélever une quantité de fluide à la pression sensiblement constante de la conduite et de réaliser un prélèvement dans un volume de taille limitée.

Le dispositif pourra comporter des moyens permettant de détecter la fin du remplissage de la chambre de puisage. Ces moyens peuvent comprendre un détecteur de la position de la membrane souple lorsque celle-ci a atteint sa déformation maximum dans la chambre, par exemple lorsque la membrane s'applique contre une paroi d'un réservoir comportant cette chambre.

L'invention sera bien comprise et ses avantages apparaîtront clairement à la lecture de quelques exemples de réalisation illustrés par les figures suivantes, parmi lesquelles :
- la figure 1 schématise l'implantation générale du dispositif selon l'invention appliquée à l'analyse sous-marine des effluents de plusieurs puits pétroliers,
- la figure 2 représente la chambre de puisage et la série d'analyseurs d'un mode de réalisation particulier du dispositif selon l'invention, et
- la figure 3 montre en détail un capteur de la boucle de pesée du dispositif selon l'invention, qui permet de mesurer le paramètre macroscopique de la qualité du fluide en écoulement.

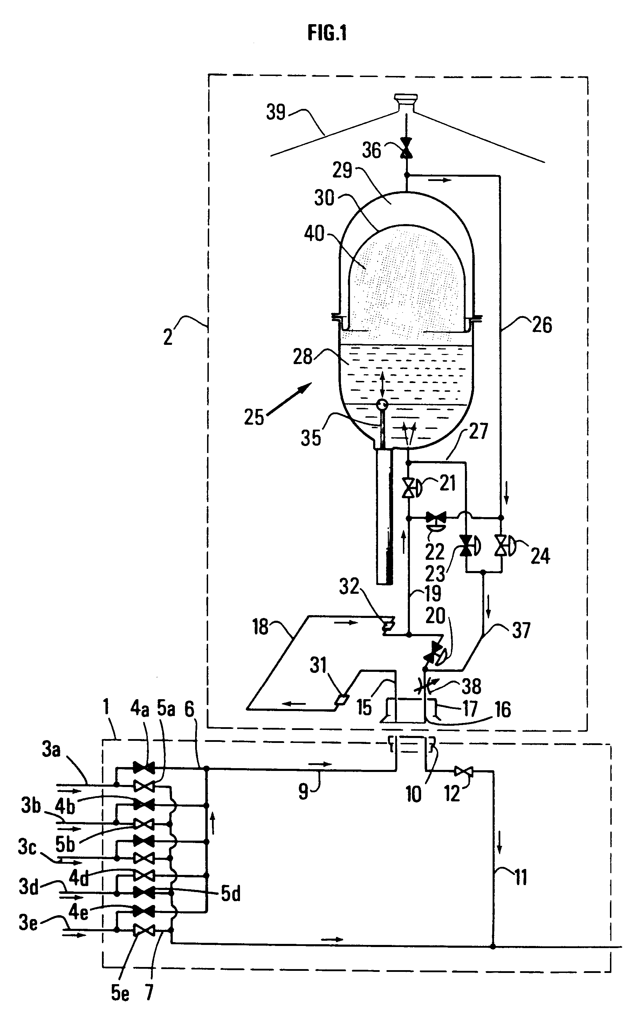

La figure 1 schématise une station sous-marine d'analyse comportant une base 1 placée sur le sol marin et un module 2 séparable surmontant cette base. La base 1 est disposée à proximité de plusieurs puits pétroliers et reliée à eux par les lignes 3a, 3b, 3c, 3d et 3e. Chacune de ces lignes 3a à 3e se termine par deux vannes repectivement 4a à 4e et 5a à 5e.

Les vannes de prélèvement 4a à 4e débouchent dans un collecteur d'échantillonnage 6 tandis que les vannes 5a à 5e débouchent dans un collecteur de production 7.

Le collecteur de production 7 concentre dans un conduit de transfert 8 les effluents non échantillonnés des différents puits. Le contrôle des vannes 4a à 4e et 5a à 5e permet de sélectionner les effluents du puits à échantillonner. L'effluent à échantillonner pénètre dans le collecteur d'échantillonnage 6, puis le quitte par la conduite 9 qui s'étend jusqu'à une embase de connecteur de pied 10. Ce connecteur de pied comporte en outre une extrémité d'une conduite 11 servant au retour des effluents échantillonnés. La conduite 11 rejoint le conduit de transfert 8 et comporte une vanne d'isolement 12.

Les conduites 9 et 11, se terminant dans l'embase du connecteur de pied 10 fixé à la base 1, sont prolongées respectivement par les conduites 15 et 16 dont les extrémités sont disposées dans une coiffe 17 du connecteur de pied fixé au module 2. La coiffe 17 coopère avec l'embase 10 pour assurer la connexion des conduites 15 et 16 avec respectivement les conduites 9 et 11.

La conduite 15, par laquelle les effluents prélevés arrivent dans le module 2, débouche à l'entrée d'une boucle de mesure densimétrique 18. La sortie de boucle densimétrique 18 est reliée à un conduit 19 et à un canal de retour sur lequel est disposé une vanne de retour 20.

Le conduit 19 est terminé par une vanne de puisage 21 contrôlant l'arrivée des effluents au réservoir 25 et par une vanne de dérivation 22 débouchant dans une conduite de compensation 26. Sur le conduit reliant la vanne de puisage 21 au réservoir 25 est branchée une conduite 27 obturable par une vanne de décharge 23 du réservoir.

Le réservoir 25 comporte une chambre de puisage 28 et une chambre auxiliaire 29 séparées l'une de l'autre par une membrane 30 souple. L'effluent prélevé est stocké dans la chambre de puisage 28 et analysé par la sonde 35.

La chambre auxiliaire 29 est reliée à la conduite de compensation 26 et à une vanne de purge 36 disposée en tête de la chambre auxiliaire 29.

La conduite de compensation 26 est terminée par une vanne de compensation 24 qui débouche, comme la vanne de décharge 23 reliée à la conduite 27, dans un conduit de retour 37 branché sur la conduite 16.

La conduite 16 comporte une duse réglable 38, permettant notamment de contrôler le débit des fluides en circulation dans la boucle densimétrique 18, soit directement lorsque la vanne de retour 20 est ouverte, soit indirectement lors du vidage de la chambre auxiliaire 29.

L'ensemble des éléments du module d'analyse 2 est protégé des chutes supérieures de matériel par un bouclier 39 qui est en outre adapté de part sa conicité à collecter les fuites polluantes éventuelles de ces éléments.

La figure 1 présente le dispositif d'analyse au cours du prélèvement des effluents sur la ligne de puits 3d. A cet effet, les vannes fermées sont représentées noircies, alors que les vannes ouvertes sont représentées éclaircies.

Pour prélever les effluents de la ligne 3d, on obture la vanne 5d et on ouvre la vanne 4d. Les effluents provenant de la ligne 3d circulent dans la conduite 9, la boucle densimétrique 18, la conduite 19, la vanne de puisage 21 avant d'entrer dans la chambre de puisage 28 dont le volume est initialement nul, la membrane 30 étant appliquée contre la paroi interne inférieure du réservoir, c'est-à-dire contre la paroi de la chambre de puisage commune avec le réservoir.

La masse du fluide située dans la boucle densimétrique 18 est déterminée par deux capteurs 31 et 32 qui mesurent la torsion de la boucle et notamment les variations de torsion dues aux variations de poids de fluide circulant dans la boucle 18.

Les vannes de retour 20, de dérivation 22 et de décharge 23 sont obturées. Le fluide, qui initialement remplit la chambre auxiliaire 29 en est vidangé par la conduite de compensation 26, et traverse : la vanne de compensation 24, le conduit de retour 37, la duse 38, la conduite 16, la vanne d'isolement 12 et la conduite de retour 11, se jetant alors dans le conduit de transfert 8.

Les chemins que parcourent les effluents du puits à analyser et le fluide de la chambre auxiliaire 29 sont indiqués sur la figure 1 par des flèches.

Lorsque la chambre de puisage 28 est remplie, c'est-à-dire lorsque son volume est sensiblement celui du réservoir 25, ou lorsque la chambre auxiliaire est vide, un capteur de position de la membrane 30 ferme la vanne de puisage 21 et ouvre la vanne de retour 20. Le volume de la chambre 28 étant connu, on mesure le temps de remplissage de la chambre 28 pour connaître le débit du puits.

Les effluents sont stockés dans la chambre 28 jusqu'à une décantation suffisante des différentes phases des effluents prélevés et la réalisation d'analyses au moyen de la sonde téléscopique 35. La sonde comporte des indicateurs de niveau, de densité, de température, de pression, de vitesse du son, de conductibilité thermique et de résistivité électrique permettant de reconnaître les différentes phases, de les caractériser et de caractériser les effluents. La sonde qui est initialement repliée, se déploie suivant la hauteur de la chambre de puisage 28 et revient dans son logement, comme indiqué par une flèche 40 sur la figure 1. Les mesures sont enregistrées en fonction de la course de la sonde 35 dans la chambre de puisage 28 et permettent après exploitation des résultats, la détermination des caractéristiques des différentes phases du fluide.

Lorsque l'analyse est terminée, la vanne 21 étant toujours fermée, on ouvre la vanne 23, on ferme la vanne 24, et on ferme la vanne 20, tout en ouvrant la vanne 22 de manière à remplir la chambre auxiliaire 29 et vider la chambre de puisage 28.

Quand la chambre auxiliaire 29 est remplie, c'est-à-dire lorsque la chambre de puisage 28 est vidée, les vannes 22 et 23 sont refermés, tandis que la vanne 20 est ouverte.

La circulation des effluents, au cours de l'analyse dans la chambre de puisage, s'effectue alors dans le module 2 par la boucle densimétrique 18, la vanne de retour 20, la duse 38 et la conduite 16.

La circulation des effluents dans la boucle densimétrique 18 avant et/ou après une analyse permet de contrôler la densité des effluents et ainsi de déterminer si l'effluent prélevé est représentatif en densité de l'effluent arrivant par la ligne 3c. Cette boucle 18 permet en outre de contrôler dans le temps la densité des effluents sans obliger leur analyse.

Pour réaliser une analyse des effluents d'une autre ligne, on ferme la vanne 4d, on ouvre la vanne 5d et on procède pour cette autre ligne de la même manière qu'avec la ligne 3d.

Sans aucun caractère limitatif quant à la taille du dispositif, le volume du réservoir 25 peut être de 10 m³ pour permettre à la fois de réaliser une analyse représentative des effluents, d'insérer du réservoir dans un module sous-marin relevable normalisé, et d'absorber dans un temps raisonnable (10 minutes) la production d'un puits de forte production, compte tenu d'un temps moyen de stabilisation (décantation de 10 minutes).

Le module peut en outre comporter une pompe pour accélérer le remplissage de la chambre 28 et permettre ainsi une étude du comportement du puits à des débits différents.

Les vannes 4a à 4e, et 5a à 5e peuvent être commandées manuellement, ou mieux, télécommandées. Les vannes 20, 21, 22, 23, 24 sont des vannes télécommandées par un opérateur ou automatisées par un programmateur.

Les vannes 12 et 36 sont des vannes manuelles. La télécommande des vannes s'effectue depuis une installation aérienne, telle une plateforme de production. Le dispositif d'analyse comporte en outre des clapets de protection des sur-pressions et sous-pressions internes et des clapets de sécurité qui ne sont pas représentés pour ne pas surcharger la figure.

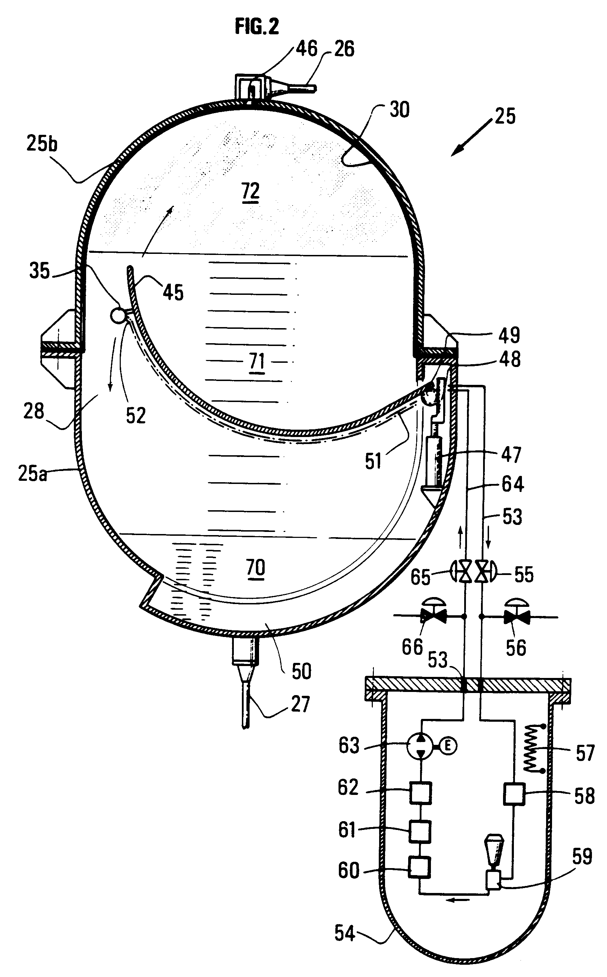

La figure 2 représente un détail d'un mode de réalisation particulier du dispositif selon l'invention comportant une série d'analyseurs 54 externes au réservoir 25 et intégrés au module d'analyse. Les références numériques des différents éléments précédemment décrits sont conservées.

Le réservoir 25 est réalisé par l'assemblage de deux cylindres au fond hémisphérique 25a et 25b, sensiblement de même volume. La membrane 30, qui est fixée au plan d'assemblage des réservoirs, épouse la paroi interne supérieure du réservoir lorsque la chambre de puisage est pleine, comme représentée à la figure 2, et épouse la paroi interne inférieure du réservoir lorsque ladite chambre est vide, le bras escamotable 45 étant rentré dans le fond inférieur du réservoir.

Le sommet du réservoir comporte un détecteur de proximité 46 de la membrane qui avertit l'opérateur humain ou le système automatique de la fin du remplissage de la chambre de puisage 28. Le bras escamotable 45 comporte à son extrémité libre la sonde 35 qui décrit sensiblement toute la hauteur du réservoir. Le mouvement du bras est réalisé par le vérin 47, tel un vérin électrique ou hydraulique, dont le piston porte une crémaillère 48 coopérant avec un pignon 49 solidaire du bras et fixé suivant son axe de rotation.

La forme du bras 45 est telle qu'une fois rentré dans son logement 50, le fond du réservoir ne présente pas d'aspérité capable de détériorer la membrane 30 alors que la chambre de puisage 28 est vide.

Au bras 45 est fixée une partie du circuit de prélèvement 51 dont une première extrémité 52 débouche au niveau de ladite sonde. La seconde extrémité 53 de ce circuit de prélèvement 51 alimente la série d'analyseurs 54 disposée dans une enceinte étanche et pressurisée à la pression atmosphérique.

La position du bras est déterminée par un capteur de position linéaire du vérin 47 ou un capteur de position angulaire du bras.

Le circuit de prélèvement comporte une vanne 55 télécommandée et un branchement normalement obturé par la vanne 56 télécommandée qui est alimenté en eau de mer pour le rinçage des analyseurs et des circuits. Le rinçage des analyseurs et des circuits peut en outre être réalisé avec l'eau décantée des effluents du puits.

L'enceinte protégeant la série d'analyseurs comporte des moyens de chauffage 57, un analyseur de conductibilité thermique 58, un analyseur de viscosité à cylindres coaxiaux, un analyseur de pression et température 59, un densitomètre vibrant 60, un pH-mètre 61, un analyseur de résistivité 62 et une pompe de circulation 63 des échantillons.

Le retour, après analyse, de l'échantillon s'effectue par le circuit 64 obturable par la vanne 65 télécommandée et débouche dans la chambre de puisage 28 sensiblement au niveau de la première extrémité 52 du circuit 51 et est de préférence orientée dans une direction opposée à celle de la première extrémité 52. Le circuit de retour 64 comporte un branchement normalement fermé par la vanne 66 télécommandée qui permet de renvoyer dans la mer l'eau de mer de rinçage, ou d'effectuer un prélèvement après analyse.

La sonde 35, ou capteur de mesure, comporte une cellule générant des ultrasons et adaptée à réaliser des mesures de vitesse du son ou de densité.

La sonde 35, notamment par la fréquence variable d'émission des ultrasons, est adaptée à la mesure dans les différentes phases, telles que l'eau 70, l'huile 71 et le gaz 72. La première extrémité 52 du circuit de prélèvement 51 est disposée à proximité de la sonde de manière que l'échantillon prélevé soit quasiment identique à celui analysé par la sonde 35.

L'enregistrement des signaux de la sonde en fonction de la position du bras permet d'évaluer la nature et le volume de chacune des phases de l'effluent prélevé dans la chambre de puisage 28.

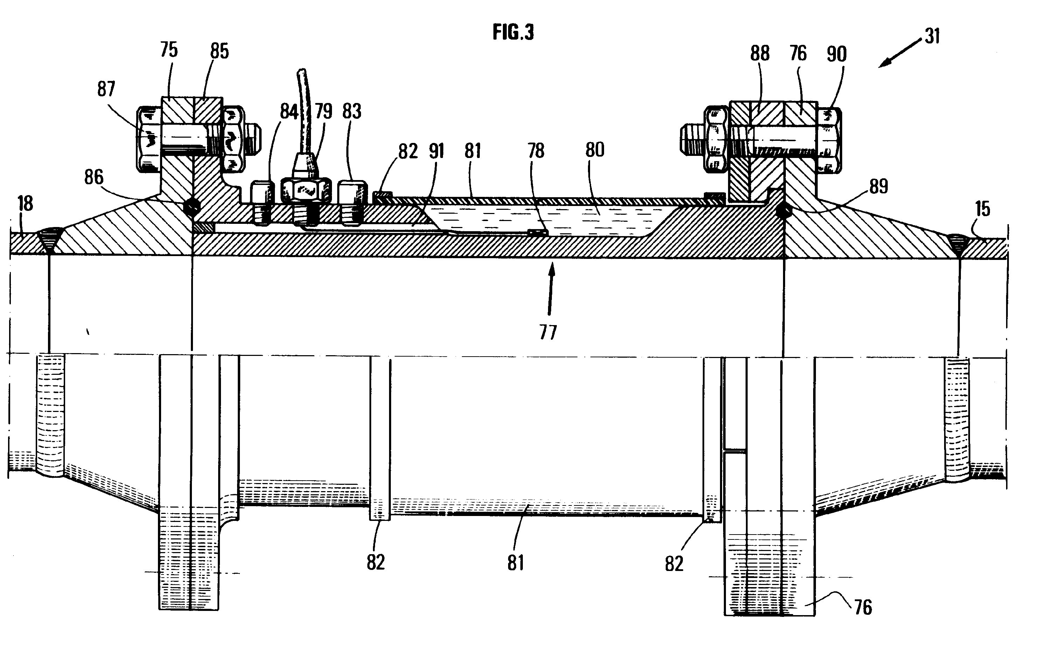

La figure 3 montre en détail un capteur 31 de la boucle de pesée ou densitomètrique du dispositif selon l'invention, tel celui de la figure 1.

Sur la figure 1, la forme en oméga (Ω) de la boucle 18 permet le positionnement du capteur de torsion à chacune de ses extrémités pour mesurer le poids de la masse du fluide dans la boucle. On aurait très bien pu utiliser d'autres formes de boucles densimétriques et mesurer non pas la torsion, mais la flexion dans une section ou l'effort tranchant est important, voire maximum. On aurait pu aussi mesurer des déformations d'une boucle dont les extrémités auraient été encastrées.

Les capteurs 31 et 32 sont disposés à chacune des extrémités de la boucle 18 qui est maintenue uniquement par ces deux capteurs. Le capteur 31 est fixé à la boucle 18 et à la conduite 15 respectivement par deux brides "API" 75 et 76 soudées à la boucle 18 et à la conduite 15.

Le capteur 31 comporte dans sa partie centrale une zone calibrée 77 sur laquelle sont disposées des jauges extensométriques 78, placées à 45° par rapport à l'axe du capteur 31, et qui mesurent la torsion du capteur. Ces jauges sont complétées, par des jauges longitudinales mesurant la flexion verticale et la traction du capteur, et par des jauges transversales mesurant la pression. L'article R1820 de Jean Avril des Techniques de l'Ingénieur, sur les capteurs extensométriques, décrit les différentes techniques de mesure utilisant les jauges extensométriques électriques. Les jauges sont reliées à une prise électrique immergeable 79, telle une prise fabriquée par Souriau et Compagnie, Jupiter ou Deutsch, fixée sur la paroi du capteur et qui peut être reliée aux instruments de mesure des déformations.

Les jauges 78 sont protégées du milieu extérieur par un fluide isolant 80 disposé à l'intérieur d'une membrane souple 81 faisant étanchéité avec le capteur 31 par un sertissage sur une portée cylindrique du capteur avec deux colliers 82. Le fluide 80 est introduit sous la membrane 81 grâce à un clapet-bouchon de remplissage 83 et un bouchon de purge 84.

Le capteur 31 comporte d'un côté une bride API 85 coopérant avec la bride 75, un joint métallique 86 du type BX et des boulons 87 pour assurer l'immobilisation et l'étanchéité avec la boucle de mesure 18, et de l'autre côté quatre demi-rondelles montées en quinconce pour constituer une bride tournante 88 qui coopèrent avec un joint métallique 89 du type BX et des boulons 90 pour assurer, sans torsion initiale, l'assemblage et l'étanchéité du capteur 31 avec la conduite 15.

## Revendications

1. Méthode d'analyse d'un fluide polyphasique en écoulement dans une conduite (9, 11, 15, 16, 18), caractérisée en ce que l'on effectue les étapes suivantes :
- on dérive ledit écoulement vers une chambre de puisage (28) pendant le temps nécessaire à un remplissage,
- la chambre (28) étant remplie, on rétablit ledit écoulement dans ladite conduite (9, 11, 15, 16, 18),
- on produit une décantation du fluide situé dans ladite chambre (28), jusqu'à obtenir une séparation des différentes phases (70, 71, 72) qui composent le fluide,
- on détermine un paramètre représentatif du volume d'au moins une phase, en mesurant une caractéristique sensible à la variation de composition d'au moins deux phases dudit fluide, et
- on contrôle un paramètre macroscopique rendant compte de la qualité du fluide dans son ensemble pendant le remplissage de la chambre de puisage et avant et/ou après ledit remplissage de ladite chambre (28).

2. Méthode selon la revendication 1, caractérisée en ce que ledit paramètre macroscopique du fluide est la masse de fluide en écoulement située dans un tronçon, tel une boucle densimétrique (18), de ladite conduite.

3. Méthode selon l'une des revendications 1 ou 2, caractérisée en ce que l'on met en oeuvre ladite méthode pour des vitesses différentes dudit écoulement.

4. Méthode selon l'une des revendications 1 à 3, caractérisée en ce que l'on prélève, dans au moins deux phases, un échantillon de substance que l'on envoie successivement vers une série d'analyseurs (54), par l'intermédiaire d'un circuit de prélèvement (51).

5. Méthode selon la revendication 4, caractérisée en ce que l'on rince le circuit de prélèvement (81) et ladite série d'analyseurs (54) en y faisant circuler de l'eau de mer.

6. Méthode selon l'une des revendications 4 ou 5, caractérisée en ce que l'on réchauffe ledit échantillon de substance prélevée et/ou de l'eau de mer avant de le faire circuler dans la série d'analyseurs (54), l'eau de mer permettant le rinçage du circuit de prélèvement de ladite série d'analyseurs ou le réétalonnage de ladite série d'analyseurs.

7. Méthode selon l'une des revendications 1 à 7, caractérisée en ce que l'on mesure le temps nécessaire pour remplir ladite chambre (28), de manière à pouvoir en déduire le débit du fluide dans ladite conduite (9, 11, 15, 16, 18).

8. Dispositif d'analyse d'un fluide polyphasique en écoulement dans une conduite (9, 11, 15, 16, 18), caractérisé en ce qu'il comporte un détecteur (31, 32) d'un paramètre macroscopique rendant compte de la qualité dans son ensemble du fluide un écoulement, ledit détecteur étant disposé sur ledit conduit en amont d'une chambre de puisage (28) adaptée à prélever une quantité du fluide circulant dans ladite conduite, ladite chambre (28) comportant des moyens de mesure (35) d'au moins un paramètre représentatif du volume d'au moins une phase, ladite conduite comportant des moyens (20, 21, 22) permettant d'orienter le fluide dans ladite chambre.

9. Dispositif selon la revendication 8, caractérisé en ce que ledit détecteur de paramètre macroscopique de la qualité du fluide est un détecteur (31, 32) de la masse de fluide en écoulement situé dans un tronçon dudit conduit.

10. Dispositif selon l'une des revendications 8 ou 9, caractérisé en ce qu'il comporte un bras mobile (45) disposé à l'intérieur de ladite chambre (28) et dont une extrémité est adaptée à balayer au moins une partie du volume de ladite chambre, ladite extrêmité du bras comportant un capteur de mesure (35) d'une caractéristique du fluide.

11. Dispositif selon la revendication 10, caractérisé en ce que ledit capteur de mesure (35) d'une caractéristique du fluide comporte une cellule générant des ultrasons.

12. Dispositif selon l'une des revendications 10 ou 11, caractérisé en ce qu'il comporte un circuit de prélèvement (51) fixé audit bras et ayant une première extrémité (52) débouchant au niveau dudit capteur de mesure (52) et une deuxième extrémité alimentant une série d'analyseurs (54).

13. Dispositif selon l'une des revendications 8 à 12, caractérisé en ce que les moyens de mesure d'un paramètre représentatif du volume d'au moins une phase de fluide comporte un capteur de mesure (35) d'une caractéristique du fluide et un capteur de position du bras.

14. Dispositif selon la revendication 12, caractérisé en ce qu'il comporte des moyens de chauffage (57) disposés à la seconde extrémité (53) du circuit de prélèvement (51).

15. Dispositif selon l'une des revendication 8 à 14, caractérisé en ce que le réservoir comporte une membrane souple (30) délimitant au moins une partie du fluide prélevé.

16. Dispositif selon l'une des revendications 8 à 15, caractérisé en ce qu'il comporte des moyens (46) permettant de détecter la fin du remplissage de ladite chambre (28).

17. Application de la méthode selon l'une des revendications 1 à 7, ou dispositif selon l'une des revendications 8 à 16 à l'analyse des effluents d'au moins un puits pétrolier.

## Patentansprüche

1. Verfahren zur Analyse eines polyphasischen Fluids, das sich im Strömungsvorgang in einer Leitung (9, 11, 15, 16, 18) befindet, dadurch gekennzeichnet, daß man die folgenden Schritte vornimmt:
- man leitet diese Strömung gegen eine Zapfkammer (28) während der für eine Füllung notwendigen Zeit um,
- nachdem die Rammer (28) gefüllt ist, stellt man diese Strömung in dieser Leitung (9, 11, 15, 16, 18) wieder her,
- erzeugt eine Dekantation des in dieser Kammer (28) befindlichen Fluids bis zum Erhalt einer Trennung der verschiedenen Phasen (70, 71, 72), die das Fluid bilden,
- bestimmt einen repräsentativen Parameter für das Volumen wenigstens einer Phase, indem man eine Charakteristik, die für die Veränderung der Zusammensetzung wenigstens zweier Phasen dieses Fluids empfindlich ist, mißt, und
- regelt einen makroskopischen Parameter, der die Qualität des Fluids insgesamt während des Füllens der Zapfkammer und vor und/oder nach diesem Füllen dieser Kammer (28) berücksichtigt.

2. Verfahren nach Anspruch 1, dadurch gekennzeichnet, daß dieser makroskopische Parameter des Fluids die Masse des im Strömungsvorgangs befindlichen Fluids, das sich in einem beschnitt, beispielsweise einer densimetrischen Schleife (18) dieser Leitung befindet, ist.

3. Verfahren nach einem der Ansprüche 1 oder 2, dadurch gekennzeichnet, daß man dieses Verfahren für unterschiedliche Geschwindigkeiten dieser Strömung durchführt.

4. Verfahren nach einem der Ansprüche 1 bis 3, dadurch gekennzeichnet, daß man in wenigstens zwei Phasen eine Substanzprobe entnimmt, die man sukzessive gegen eine Reihe von Analysatoren (54) vermittels eines Entnahmekreises (51) schickt.

5. Verfahren nach Anspruch 4, dadurch gekennzeichnet, daß man den Entnahmekreis (81) und diese Reihe von Analysatoren (54) durchspült, indem man hierin Meereswasser zirkulieren läßt.

6. Verfahren nach einem der Ansprüche 4 oder 5, dadurch gekennzeichnet, daß man diese entnommene Substanzprobe und/oder die Meereswasserprobe erwärmt, bevor man in der Reihe von Analysatoren (54) das Meereswasser zirkulieren läßt, welches das Spülen des Entnahmekreises dieser Reihe von Analysatoren oder das Nacheichen dieser Reihe von Analysatoren erlaubt.

7. Verfahren nach einem der Ansprüche 1 bis 6, dadurch gekennzeichnet, daß man die Zeit mißt, die zur Füllung dieser Kammer (28) notwendig ist, derart, daß man hieraus den Fluiddurchsatz in dieser Leitung (9, 11, 15, 16, 18) ableiten kann.

8. Analysevorrichtung für ein polyphasisches in einer Leitung (9, 11, 15, 16, 18) im Strömungsvorgang befindliches Fluid, dadurch gekennzeichnet, daß sie einen Detektor (31, 32) eines makroskopischen Parameters umfaßt, der die Qualität des insgesamt im Strömungsvorgang befindlichen Fluids berücksichtigt, wobei dieser Detektor an dieser Leitung vor einer Zapfkammer (28) angeordnet ist, die ausgelegt ist, um eine Menge des in dieser Leitung zirkulierenden Fluids zu entnehmen, wobei diese Kammer (28) Meßmittel (35) für wenigstens einen für das Volumen wenigstens einer Phase repräsentativen Parameter umfaßt, und diese Leitung Mittel (20, 21, 22) umfaßt, die es ermöglichen, das Fluid in dieser Kammer zu orientieren.

9. Vorrichtung nach Anspruch 8, dadurch gekennzeichnet, daß dieser Detektor für den makroskopischen Parameter der Fluidqualität ein Detektor (31, 32) der im Strömungsvorgang befindlichen Fluidmasse, der in einem Abschnitt dieser Leitung angeordnet ist, ist.

10. Vorrichtung nach einem der Ansprüche 8 oder 9, dadurch gekennzeichnet, daß sie einen beweglichen Arm (45) umfaßt, der im Inneren dieser Kammer (28) angeordnet ist und von dem ein Ende so ausgelegt ist, daß es wenigstens einen Teil des Volumens dieser Rammer bestreicht, wobei dieses Ende des Arms einen Meßwertaufnehmer (35) für eine Charakteristik des Fluids umfaßt.

11. Vorrichtung nach Anspruch 10, dadurch gekennzeichnet, daß dieser Meßwertaufnehmer (35) für eine Fluidcharakteristik eine ultraschallerzeugende Zelle umfaßt.

12. Vorrichtung nach einem der Ansprüche 10 oder 11, dadurch gekennzeichnet, daß sie einen Entnahmekreis (51) umfaßt, der an diesem Arm befestigt ist und ein erstes Ende (52) hat, das in Höhe dieses Meßwertaufnehmers (52) mündet und über ein zweites Ende verfügt, das eine Reihe von Analysatoren (54) speist.

13. Vorrichtung nach einem der Ansprüche 8 bis 12, dadurch gekennzeichnet, daß die Mittel zum Messen eines für das Volumen wenigstens einer Fluidphase repräsentativen Parameters einen Meßwertaufnehmer (35) für eine Charakteristik des Fluids sowie einen Positionsmeßwertaufnehmer des Arms umfaßt.

14. Vorrichtung nach Anspruch 12, dadurch gekennzeichnet, daß sie Heizmittel (57) umfaßt, die am zweiten Ende (53) des Entnahmekreises (51) angeordnet sind.

15. Vorrichtung nach einem der Ansprüche 8 bis 14, dadurch gekennzeichnet, daß der Speicher eine nachgiebige Membran (30) umfaßt, die wenigstens einen Teil des entnommenen Fluids begrenzt.

16. Vorrichtung nach einem der Ansprüche 8 bis 15, dadurch gekennzeichnet, daß sie Mittel (46) umfaßt, die das Ende des Füllens dieser Rammer (28) erfassen.

17. Anwendung des Verfahrens nach einem der Ansprüche 1 bis 7 oder der Vorrichtung nach einem der Ansprüche 8 bis 16 auf die Analyse der Abströme aus wenigstens einem Erölbohrloch.

## Claims

1. Method of analysing a multiphase fluid flowing in a pipe (9, 11, 15, 16, 18), characterised in that the following stages are carried out:
- the said flow is tapped off into a sump (28) for the length of time necessary for filling,
- once the sump (28) is full, the said flow is re-started in the said pipe (9, 11, 15, 16, 18),
- the fluid in the said sump (28) is decanted for a certain length of time until the various phases (70, 71, 72) making up the fluid, separate,
- a parameter representing the volume of at least one phase is determined by measuring one characteristic sensitive to the variation in the composition of at least two phases of the said fluid, and
- a macroscopic parameter is monitored which gives information on the quality of the fluid as a whole during the filling of the sump and before and/or after the said filling of the said sump (28).

2. Method according to Claim 1, characterised in that the said macroscopic parameter of the fluid is the weight of fluid flowing in one section, such as a densimetric loop (18) of the said pipe.

3. Method according to one of Claims 1 or 2, characterised in that the said method is implemented for different rates of the said flow.

4. Method according to one of Claims 1 to 3, characterised in that a sample of substance is taken from at least two phases, which is sent successively to a series of analysers (54) through a sampling circuit (51).

5. Method according to Claim 4, characterised in that the said sampling circuit (81) and the said series of analysers (54) are rinsed by circulating sea water through them.

6. Method according to one of Claims 4 or 5, characterised in that the said sample of substance and/or sea water is heated before passing it through the series of analysers (54), the sea water enables the sampling circuit of the said series of analysers to be rinsed or the said series of analysers to be recalibrated.

7. Method according to one of Claims 1 to 7, characterised in that the time required for filling the said sump (28) is measured so that the flow rate of the fluid in the said pipe (9, 11, 15, 16, 18) can be deduced.

8. Device for analysing a multiphase fluid flowing in a pipe (9, 11, 15, 16, 18), characterised in that it includes a detector (31, 32) of a macroscopic parameter giving information on the overall quality of the flowing fluid, the said detector being disposed in the said pipe upstream of a sump (28) designed to sample a quantity of the fluid passing through the said pipe, the said sump (28) having means (35) for measuring at least one parameter representing the volume of at least one phase, the said pipe having means (20, 21, 22) allowing the fluid to be oriented in the said sump.

9. Device according to Claim 8, characterised in that the said detector of a macroscopic parameter for the quality of the fluid is a detector (31, 32) of the weight of flowing fluid located in a section of the said pipe.

10. Device according to one of claims 8 or 9, characterised in that it includes a movable arm (45) disposed inside the said sump (28), one end of which is designed to sweep at least part of the volume of the said sump, the said end of the arm having a sensor (35) for measuring one characteristic of the fluid.

11. Device according to Claim 10, characterised in that the said sensor (35) measuring one characteristic of the fluid has a cell generating ultrasound.

12. Device according to one of Claims 10 or 11, characterised in that it includes a sampling circuit (51) attached to the said arm and having a first end (52) terminating at the said measuring sensor (52) and a second end supplying a series of analysers (54).

13. Device according to one of Claims 8 to 12, characterised in that the means for measuring a parameter representative of the volume of at least one fluid phase has a sensor (35) measuring one characteristic of the fluid, and a sensor detecting the position of the arm.

14. Device according to Claim 12, characterised in that it includes heating means (57) disposed at the second end (53) of the sampling circuit (51).

15. Device according to one of Claims 8 to 14, characterised in that the reservoir has a flexible diaphragm (30) delimiting at least part of the fluid sampled.

16. Device according to one of Claims 8 to 15, characterised in that it has means (46) for detecting the end of filling of the said sump (28).

17. Application of the method according to one of Claims 1 to 7 or the device according to one of Claims 8 to 16, to the analysis of the effluents of at least one oil well.
